Europäisches Patentamt

European Patent Office

Office européen des brevets

(19)

(11) Publication number: **0 642 023 A1**

## (12) EUROPEAN PATENT APPLICATION
## published in accordance with Art. 158(3) EPC

(21) Application number: **92920915.3**

(22) Date of filing: **02.10.92**

(86) International application number:
**PCT/JP92/01276**

(87) International publication number:
**WO 93/07488 (15.04.93 93/10)**

(51) Int. Cl.6: **G01N 33/576**

(30) Priority: **02.10.91 JP 255524/91**
**26.03.92 JP 68695/92**

(43) Date of publication of application:
**08.03.95 Bulletin 95/10**

(84) Designated Contracting States:
**AT BE CH DE ES FR GB IT LI LU NL**

(71) Applicant: **EIKEN KAGAKU KABUSHIKI KAISHA**
**33-8, Hongo 1-chome**
**Bunkyo-ku,**
**Tokyo 114 (JP)**
Applicant: **The Research Foundation for Microbial Diseases of Osaka University**
**3-1 Yamadaoka**
**Suita-shi**
**Osaka (JP)**
Applicant: **TANABE SEIYAKU CO., LTD.**
**2-10, Dosho-machi 3-chome**
**Chuo-ku,**
**Osaka-shi,**
**Osaka-fu (JP)**

(72) Inventor: **ISHIBASHI, K., Immunochemical Research Lab.**
**Eiken Kagaku K K**
**1381-3, Azahigashiyama,**
**Shimoishigami**
**Otawara-shi**
**Tochigi329-26 (JP)**
Inventor: **ITOH,M., Immunochemical Research**

Lab.
**EikenKagakue K K**
**1381-3, Azahigashiyama,**
**Shimoishigami**
**Otawara-shi**
**Tochigi 329-26 (JP)**
Inventor: **YOSHIDA, I., Kanonji Inst., The Res. Found. for**
**Microb. Diseases of Osaka Univ.**
**9-41**
**Yahata-cho**
**2-ch2-chome,**
**Kanonji-shi, Kagawa 768 (JP)**
Inventor: **TAKAMIZAWA, A., Kanonji Inst., The Res. Found.for**
**Microb. Diseases of Osaka Univ.**
**9-41,**
**Yahata-cho**
**2-chome,**
**Kanonji-shi, Kagawa 768 (JP)**
Inventor: **SHIBATANI, T., Research Lab. of Applied Biochem.**
**Tanabe Seiyaku Co., Ltd.**
**16-89, Kashima3-chome**
**Yodmogawa-ku, Osaka-shi**
**Osaka 532 (JP)**

(74) Representative: **Greaves, Carol Pauline et al**
**Mewburn Ellis**
**York House**
**23 Kingsway**
**London WC2B 6HP (GB)**

(54) **REAGENT AND METHOD FOR TESTING HEPATITIS C.**

㊗ A reagent for testing an antibody of hepatitis C by using as an antigen a peptide containing an essential peptide comprising a specified sequence having an epitope of an HCV structural region, Arg *Xaa* Gly Pro Arg Leu Gly Arg Arg Pro, wherein Xaa represents preferably Leu, Lys or Arg. The reagent serves to detect an antibody against the HCV structural region specifically at a high detection rate, whereby HCV infection can be tested accurately in an early stage. The necessary peptide can be chemically synthesized readily.

FIELD OF THE INVENTION

The present invention relates to reagents for testing for hepatitis C and a method of testing for the same. According to recent studies, the number of patients suffering from non-A non-B hepatitis is estimated to be about 2 million in Japan, of which 90-95% are hepatitis C.

BACKGROUND ART

The molecular structure of the hepatitis C virus (hereinafter referred to as HCV) which is responsible for hepatitis C is being clarified; in recent years, several reagents to detect the presence or absence of antibodies against HCV (HCV antibodies) have become commercially available.

As to HCV markers which have been put in practical use to date, C-100-3 antigen corresponding to a part of NS-3 to 4, which is the non-structural region of HCV, is known (Science 244, 359-362, 1989). The use of the C-100-3 antigen as the HCV marker makes it possible to analyze antibodies against HCV for the first time ever. However, when subjects who were infected with HCV were actually tested using the C- 100-3 antigen, a problem remained; that is, only about 70% of the subjects tested HCV positive and the remaining 30% tested false negative. On the other hand, it was reported that when an ELISA kit for detecting HCV antibodies containing the C-100-3 antigen was actually used for screening blood for transfusion, as much as 1.5% of the blood tested positive, out of which nearly half showed false positive.

Later, in order to improve HCV antibody detectability and to detect HCV in an early stage of infection, the use of the HCV core region as an antigen was proposed. Several kits for detection of HCV antibodies using the HCV core region protein as an antigen have been studied; these kits are called second generation kits against the above-mentioned kits in which the C-100-3 antigen is used. An attempt was made, wherein one synthesized peptide corresponding to an epitope of the core region protein combined with a synthetic peptide corresponding to two epitopes in the above-mentioned non-structural region of HCV, i.e., NS-3 to 4, in order to detect HCV antibodies (Proc. Natl. Acad. Sci. USA 88, 3647-3651, 1991). The specificity of detection by this method was better than that with the use of the C-100-3 antigen as an HCV marker; however, since only one epitope in the core region was selected, antibodies produced against epitopes other than this epitope could not be detected, which resulted in insufficient detectability for antibodies against HCV.

Several epitopes which react with antibodies against the core region protein have been disclosed in Japanese Patent Laid-Open No. 139282/1991 although they have not been put in practical use. If a multiple number of these epitopes are utilized, antibodies detectability may be increased; however, decreased specificity is anticipated and, in addition, the method is economically disadvantageous.

Further, since the peptides used in detecting HCV antibodies to date were primarily obtained by genetic recombination, non-specific reactions tended to occur. For example, since the above-mentioned C-100-3 antigen was a part of a product of genetic recombination in which the antigen was expressed as a protein fused with superoxide dismutase (referred to as SOD hereafter), HCV antibodies cannot be effectively detected by this antigen unless an additional test using the immuno-blot technique or the like is carried out to check a false positive reaction caused by the reaction with the SOD component.

Moreover, when a fused protein containing ARIMA #14 (to be described later), which is partially homologous to the HCV core region and was obtained by the expression using T-7 phage vector, was used as an antigen, a false positive reaction was caused by antibodies against T-7 phage proteins. In this case, the false positive reaction can be eliminated by absorbing the non-specific reactions using the T-7 phage proteins.

Thus, in the case where peptides obtained by genetic recombination are used as antigens for detection of antibodies, special countermeasures against non-specific reactions is necessary. Moreover, at present, particular measure is required to solve the problem of these non-specific reactions and the problem has not been effectively solved. Therefore, development of antigens which are highly sensitive to HCV antibodies and at the same time maintain sufficient specificity is desired. Apart from the problem of non-specific reactions which are attributable to the above-mentioned fused proteins, there is a fundamental problem in terms of antigen-antibody reactions. That is, as mentioned above, the use of a single epitope of the HCV core region results in relatively low HCV detectability while the use of a multiple number of epitope improves the detectability but increases non-specific reactions. In general, in detecting specific antibodies using a certain antigen, if an antigen having high reactivity is used to increase detectability, non-specific reactions increase with the increase in detectability and thus specificity decreases (namely, overmeasured); in contrast, if an antigen having high specificity is used, the number of detectable antibodies decreases (namely, undermeasured). However, no suitable antigen to meet these contradictory conditions is known at this time.

DISCLOSURE OF THE INVENTION

In view of actual situation of the prior art, an objective of the present invention is to provide reagents for testing for HCV antibodies, which are highly practical and improve HCV antibody detectability but do not sacrifice specificity despite the fact that only a small number of species of peptides are used and the number of amino acids composing the peptides is small, and also to provide a method for testing for the same.

Such an objective can be accomplished by the following means. That is, the present invention includes reagents for testing for hepatitis C, which contain one or more kinds of peptides having
Sequence 1: Arg Xaa Gly Pro Arg Leu Gly Arg Arg Pro (wherein Xaa is any given amino acid),
or contain multiple peptides in which peptides having the following Sequence 2 and/or Sequence 3 are mixed with the peptide(s) of Sequence 1:
Sequence 2: Gln Arg Lys Thr Lys Arg Ser Thr Asn Arg Arg
Sequence 3: Thr Gln Gln Arg Lys Thr Lys Arg Ser Thr Asn Arg Arg Arg Ser Lys Asn Glu Lys.

Further, the present invention includes a method for testing for hepatitis C by detecting antibodies which are reactive to one or more kinds of peptides having
Sequence 1: Arg Xaa Gly Pro Arg Leu Gly Arg Arg Pro (wherein Xaa is any given amino acid),
or multiple peptides in which peptides having the following Sequence 2 and/or Sequence 3 are mixed with the peptide(s) of Sequence 1:
Sequence 2: Gln Arg Lys Thr Lys Arg Ser Thr Asn Arg Arg
Sequence 3: Thr Gln Gln Arg Lys Thr Lys Arg Ser Thr Asn Arg Arg Arg Ser Lys Asn Glu Lys.

Here, abbreviations for each amino acid are as follows:

| | |
|---|---|
| Ala: Alanine | Leu: Leucine |
| Arg: Arginine | Lys: Lysine |
| Asn: Asparagine | Met: Methionine |
| Asp: Aspartic acid | Phe: Phenylalanine |
| Cys: Cystine | Pro: Proline |
| Glu: Glutamic acid | Ser: Serine |
| Gln: Glutamine | Thr: Threonine |
| Gly: Glycine | Trp: Tryptophan |
| His: Histidine | Tyr: Tyrosine |
| Ile: Isoleucine | Val: Valine |

Among the 10 amino acids in Sequence 1, Xaa is any given amino acid, which can be selected from a relatively wide range. Other amino acids in the sequence have to be specified in order to obtain high antigenicity but the Xaa amino acid can vary to a certain extent. For example, Xaa in a partial sequence of the HCV core region disclosed in Japanese Patent Laid-Open No. 139282/1991, which corresponds to Sequence 1, is Leu; however, Xaa in Sequence 1 according to the present invention is not limited to Leu and high antigenicity can also be attained with Lys or Arg. In fact, Lys is more preferable than Leu.

Although the amino acid sequences of the peptides given by Sequences 1, 2 and 3 in the present invention are considerably shorter than known sequences (for example, those described in Japanese Patent Laid-Open No. 139282/1991), they exhibit substantially antigenic functions in the same manner as one to three epitope components in the HCV core region. Namely, reconciliation of specificity and reactivity, which is the fundamental objective in the method of detection by means of an antigen-antibody reaction, can be attained by using peptides, as antigens, corresponding to one specified partial sequence (corresponding to Sequence 1), two specified partial sequences (corresponding to Sequence 1 + Sequence 2 or 3) or three specified partial sequences (corresponding to Sequences 1 + 2 + 3 ) of epitope structure in the HCV core region. In this method, non-specific reactions can be suppressed because no fused protein is used.

As clearly demonstrated in the results of Examples described below, the reagents for detecting HCV antibodies according to the present invention are excellent in their detectability and specificity.

Further, the peptides having Sequence 1 according to the present invention can be obtained by chemical synthesis because the number of amino acids in the compositions is relatively small, so that non-specific reactions which frequently occur in peptide antigens produced by genetic recombination can be eliminated. Moreover, the present invention is economically advantageous because sufficiently high detec-

4

tability can be achieved using as few as one or two kinds of peptides.

In addition, reactivity of the peptides according to the present invention can be improved by introducing a cross-linking agent such as Sulfo-SMCC therein. If a serum protein such as human serum albumin (HSA) is bound as a vehicle protein via the introduced cross-linking agent, it is possible to improve reactivity and at the same time to suppress non-specific reactions.

## BEST MODE FOR CARRYING OUT THE INVENTION

The present invention will be explained more in detail as follows. As to the peptides required in the present invention, chemically synthesized peptides can be used. The peptides containing Sequence 1 include those in which plural (up to about 20) amino acids are optionally added to Sequence 1 and those which are modified by spacers or vehicle proteins as described below. The optional amino acids can be added to Sequence 1 either at the N-terminal or C-terminal of Sequence 1. For example, the amino acid sequences can be selected referring to several known sequences of the HCV core region adjoining to sequences corresponding to Sequence 1; however, they are not limited to those sequences. Other examples of the peptides include those in which the peptide of Sequence 2 or Sequence 3 is bound to the peptide of Sequence 1. In binding the peptide of Sequence 2 or Sequence 3 to the peptide of Sequence 1, it is preferable to bind them via an arbitrary peptide having plural (up to about 20) amino acids in order to maintain reactivity. For certain, it is also possible to construct the present invention using the peptide having Sequence 1 only.

Next, if the peptides of Sequence 1 and Sequence 2 and/or Sequence 3 are used as antigens in combination, namely in a mixed form without being linked together, these peptides give better results in detectability than the peptide of Sequence 1 only or other peptides containing Sequence 1. This is because detectability occasionally decreases when one kind of peptide is solely used. When peptides are used in combination, a preferable ratio for the amount of the peptide of Sequence 1 to the peptides of Sequence 2 and/or Sequence 3 may be in the range approximately between 1:2 and 2:1. Further, when the peptides of Sequence 1 and Sequence 2 and/or 3 are used in combination, they are each bound to a vehicle protein, another optional peptide or the like and then mixed together for use, as will be subsequently explained. Xaa in Sequence 1 can be arbitrarily selected and the peptides of Sequence 1 with different amino acids for Xaa are considered to have different epitopes; however, it is not known that these epitopes are distinctly different from one another since the amino acid sequence except for Xaa are the same. Therefore, the use of the peptide of Sequence 1 and the peptide of Sequence 2 in combination, which are clearly different from each other in terms of corresponding epitopes, results in better detectability than the use of a multiple number of different peptides of Sequence 1. The use of multiple kinds of peptide of Sequence 1 is, however, not necessarily eliminated from the present invention and may be effective in certain cases.

The peptide molecules in the present invention can be applied to the antigen-antibody reaction without modification; however, their reactivity can be improved by linking them to a spacer molecule or a vehicle protein. For example, reactivity with antibodies can be improved by covalently binding a spacer such as sulfosuccinimidyl-4-(N-maleimidomethyl)cyclohexane-1-carboxylate (hereinafter referred to as Sulfo-SMCC), sulfosuccinimidyl-6-[3-(2- pyridyldithio)propionamido]hexanoate (hereinafter referred to as Sulfo-LC-SPDP) and N-(epsilon- maleimidocaproiloxy)succinimido (hereinafter referred to as EMCS) to the peptides at the N-terminal thereof.

If an inactive vehicle protein such as human serum albumin (hereinafter referred to as HSA) or bovine serum albumin (hereinafter referred to as BSA) is further bound via the spacer, better results can be obtained. In binding a vehicle protein, a spacer can be introduced at the vehicle protein site as well as the peptide site. As a vehicle protein, a protein derived from the same animal species that is to be tested is preferably used in order to minimize non-specific reactions as much as possible. Namely, if human serum is a sample to be tested, HSA is preferably used.

It is possible to utilize the peptides according to the present invention, or the modified peptides thereof, in combination with an appropriate immunoassay system. For example, in the case of the solid phase immunoassay in which antigens are bound to the surface of beads or container walls to form a solid phase for the reaction with antibodies to be detected, the peptides of the present invention or the modified peptides thereof are bound to form a solid phase by physical adsorption or chemical bonding. When a multiple species of peptides are used, they may be bound onto a vehicle as a mixture, or each species may be separately bound to a vehicle and then mixed. In this case, HCV antibodies caught by the peptides on the solid phase can be detected by using antibodies against human immunoglobulins (a sandwich immunoassay). If the antibodies against human immunoglobulins recognizing different classes are used for the assay, the detected HCV antibodies can be classified. In addition to the sandwich immunoassay, a competitive method using labeled antibodies can be applied as an immunoassay system in which peptides bound to the solid phase are used.

In the present invention, since the number of epitopes is in fact at most two or three, it is possible to apply monoclonal antibodies as labeled antibodies when the competitive method is used. Monoclonal antibodies which are specific to the peptides to be used can be applied as the labeled antibodies in an appropriate mixture according to the mixing ratio and affinity of the peptides on the solid phase.

The peptides of the present invention or the modified peptides thereof can be used also as the labeled antigens. In this case, the immunoassay system can be constructed in combination with the method in which antibodies against human immunoglobulins are made into solid phase (the sandwich method) or with the method in which antibodies against HCV are made into a solid phase (the competitive method). Labeling in the various kinds of immunoassay systems mentioned above can be carried out by known methods using enzymes, luminescent materials, fluorescent materials, radioisotopes or the like. These labeling materials are bound using spacer molecules, avidin-biotin bonds or the like.

Further, peptides according to the present invention or the modified peptides thereof can be applied to the particle agglutination reaction or the agglutination inhibiting reaction in combination with latex particles or the like.

HCV test reagents according to the present invention and components necessary for the test, which include diluents, substrates for label detection and the positive control, can be appropriately combined and made into a kit. For example, the peptides according to the present invention can be bound to solid phase beads to make an assay kit combined with labeled anti-human IgG antibodies.

The peptides which contain Sequence 1 according to the present invention have high reactivity with HCV antibodies and cause little non-specific reactions with other antibodies. By incorporating peptides having Sequence 2 and/or Sequence 3 into these peptides, detectability of HCV antibodies can be improved. The peptides according to the present invention have sequences in common with partial or complete sequences of epitope structures disclosed, for example, in Japanese Patent Laid-Open No. 139282/1991. However, since the peptides of the present invention are considerably shorter than those disclosed in Japanese Patent Laid-Open No. 139283/1991, they can readily be synthesized. Further, since the specific peptides are selected so as to maintain high specificity against HCV antibodies, highly specific analyses can be accomplished.

The present invention will be explained in detail in the following Examples.

[1] Peptide synthesis

Six peptides having the following amino acid sequences according to the present invention were synthesized using a peptide synthesizer (a product of Biosearch, Model 9600. In this regard, Sequence 1 in which Xaa is Leu, Lys or Arg is referred to as Sequence 1a, 1b or 1c, respectively. A mark (-) indicates a binding site of Sequence 1 with other amino acids.

Sequence 1a:
Arg Leu Gly Pro Arg Leu Gly Arg Arg Pro

Sequence 2:
Gln Arg Lys Thr Lys Arg Ser Thr Asn Arg Arg

Sequence 3:
Thr Gln Gln Arg Lys Thr Lys Arg Ser Thr Asn Arg Arg Arg Ser Lys Asn Glu Lys

Sequence 1a7 (Sequence 1a + 7 amino acids):
Thr Arg-Arg Leu Gly Pro Arg Leu Gly Arg Arg Pro -Ala Leu Met Ala Val

Sequence 1b9 (Sequence 1b + 9 amino acids):
Val Val Pro-Arg Lys Gly Pro Arg Leu Gly Arg Arg Pro-Ala Leu Met Ala Val Lys

Sequence 1c9 (Sequence 1c + 9 amino acids):
Pro Thr Arg-Arg Arg Gly Pro Arg Leu Gly Arg Arg Pro-Ala Leu Met Ala Val Lys

The synthesis was carried out according to the stepwise solid phase method. t-Butyloxycarbonylaminoacyl-4- (oxymethyl)-Merrifield resin (a product of Watanabe Kagaku Kogyo) was used as a solid phase and amino groups of amino acids (L-form) are protected by t-butyloxycarbonyl groups (hereinafter referred to as t-BOC). Furthermore, side chains of each amino acid were protected by the following protective groups:

Threonine (Thr/T), Serine (Ser/S): o-benzyl group

Glutamine (Glu/E): gamma-benzyl ester

Arginine (Arg/R): mesitylene-2-sulfonyl group

Lysine (Lys/K): 2-chlorobenzyloxycarbonyl group

Side chain of other amino acids: unprotected

Coupling was carried out by treating with trifluoroacetic acid to remove protection, the t-BOC group, of t-BOC amino acid at the terminal of the peptide on a resin, activating the carboxyl group of the amino acid next to the amino acid to be coupled using N,N'-diisopropylcarbodimido and mixing to react at room temperature for 1-4 hours. The reaction time was determined according to the kinds and combinations of amino acids. Free amino acids which remained intact were inactivated by acetylation with 1-acetylimidazol.

After completion of the necessary reaction process, protective groups were removed and synthesized peptides were excised from the resin using trimethylsilyltrifluoromethanesulfonic acid, thioanisole and trifluoroacetic acid and the peptides were recovered with ether. The peptides thus recovered were prepurified by gel filtration and then subjected to reversed-phase HPLC on an ODS-80TM (water-acetonitrile, containing 0.1% TFA) column and the peak fraction was fractionated.

[2] Preparation of test reagents - 1

Using the peptides synthesized in [1], 5 different reagents for detecting HCV antibodies were prepared. Reagent numbers and corresponding sequences were as follows:

Reagent 1a: Sequence 1a

Reagent 2: Sequence 2

Reagent 3: Sequence 3

Reagent 1a7: Sequence 1a7

Reagent 1a7*2: Sequence 1a7 + Sequence 2

Reagent 2 and reagent 3 were prepared to investigate the effect of the single use of each peptide of Sequence 2 and Sequence 3, thereby the effect of the reagents according to the present invention being verified by comparison. The peptides of Sequences 1a, 1a7, 2 and 3 were made into a solid phase according to the following method.

Each synthesized peptide antigen was dissolved in a 50 mM carbonate buffer solution (pH 9.6) at a concentration of 2.0 micrograms/ml (concentrations ranging from about 2.0 to 5.0 micrograms/ml resulted in similar detectability) and the resultant solution was dispensed in 100 microliter portions in each well of a microtiter plate for ELISA (MAXISORP C12, a product of Nunc; the amount of antigen was 0.2 microgram per well; the same hereinafter). The plate was allowed to stand at 4°C overnight and each well was washed three times with 300 microliters of a washing solution (a 10 mM phosphate buffer solution containing 0.05% Tween 20, pH 7.2) per wash. After drying the plate, the reagents corresponding to the individual sequences were obtained as a solid phase. The peptides of Sequence 1a7 and Sequence 2 were mixed in a 1:1 ratio and adsorbed onto the microtiter plate (0.2 + 0.2 = 0.4 microgram/well) in the same manner to obtain a reagent as a solid phase.

Meanwhile, the anti-human immunoglobulin G mono-clonal antibody was treated by the maleimido method to prepare the peroxidase-labeled antibody (in a phosphate buffer solution containing 0.1% BSA; hereinafter referred to as POD- labeled anti-IgG antibody). A substrate solution for color development comprising 0.17 mg/ml tetramethylbenzidine, 0.015% hydrogen peroxide and 0.125 M citrate buffer (pH 4.2) was also prepared.

[3] Detection of HCV antibodies - 1

Human serum samples (20 specimens) which were suspected to be infected with HCV and normal human serum samples (10 specimens) were subjected to ELISA analysis using reagents 1a, 1a7, 2, 3 and 1a7*2. For comparison, reagents prepared from known peptides, B-1 and ARIMA #14 (hereinafter referred to as N-14) were also subjected to the analysis. In this regard, the antigen concentration for B-1 and N-14 was 0.1 microgram/well. This antigen concentration was different from those used for the above-mentioned Sequence 1a or the like, which was 0.2 microgram/well, because the optimum concentrations (economical concentrations not resulting in a decrease in sensitivity) is different for each antigen so that the optimum conditions for individual antigens were accordingly selected.

B-1 is a peptide corresponding to the amino acid sequence from position 10 to 122 counting from the N-terminal of the amino acid sequence of the HCV structural region described in J. Virology, 65/3, 1105, 1991. ARIMA #14 is a peptide which was reported as an epitope in the HCV structural region (BIOmedica, 6(2), 26, 1991). Further, comparison was also made with results of analysis using a commercial kit for HCV antibody detection (in which C-100-3 antigen was used). Furthermore, part of the serum specimens were subjected to a confirmation test using a commercial kit for a supplementary test by the immuno-blot technique (RIBA Test, a product of Ortho Pharmaceuticals Corp.). The test was carried out in the following manner.

200 microliters of a solution for sample dilution (0.05% Tween 20, 0.125% NaN$_3$, in 10 mM phosphate buffer, pH 7.2) and 20 microliters of serum sample were placed into each of the wells of the plate, which were coated with the antigen, and the reaction was allowed to proceed at 25ºC for 1 hour. After the reaction, the reaction solution was removed and each well was washed 5 times with 300 microliters of a washing solution (0.05% Tween 20, 10 mM phosphate buffer, pH 7.2) at a time and then the POD-labeled anti-IgG antibody was dispensed in 100 microliter portions per well and the reaction was allowed to proceed at 25ºC for 1 hour. After the reaction, the reaction solution was removed, each well was washed 5 times with 300 microliters of the washing solution per wash. Color development was then allowed to proceed at 25ºC for 30 minutes by the addition of 100 microliters of the color developing substrate solution to each well. After terminating the reaction by the addition of 2 N sulfuric acid, the optical densitie of each well at 450 nm was determined. Results are shown in Table 1 (in the Table, reagents are designated with P). Table 1 indicates that as compared with the results obtained by the supplementary test using the immuno-blot technique (designated with "RIBA in the Table), a considerable number of positive reactions were observed using the commercial kit (designated with "C-100" in the Table) while the results obtained by using reagent 1a7 and reagent 1a7*2 were well consistent with those obtained by using RIBA; thus the high detectability of reagent 1a7 and reagent 1a7*2 was proved. Further, reagent 2 and reagent 3 were confirmed to be similarly reactive, but they failed to detect some positive samples in contrast to reagents having sequence 1, such as 1a, 1a7 or 1a7*2 (see samples No. 16 and 17), which indicates that positive samples might occasionally be left undetected with the single use of the peptide of Sequence 2 or 3. Furthermore, it was indicated that improvement in the HCV detectivity was better with reagent 1a7*2 than reagent 1a or 1a7. Although whether the results were consistent with those using B-1 or not may not directly imply superiority or inferiority of the reagents for testing, reagents which showed negative reaction for samples suspected to be infected with HCV and positive when B-1 was used are considered to have low detectability in general. Judging from this criterion, reagents 1a, 1a7 and 1a7*2 were superior to reagents 2, 3 and N-14.

Table 1

| | No | P1a7 | P1a | P2 | P3 | P1a7*2 | B-1 | N-14 | C-100 | RIBA |
|---|---|---|---|---|---|---|---|---|---|---|
| Samples suspected to be infected with HCV | 1 | 1.237 + | 1.173 + | 1.928 + | 1.882 + | 1.933 + | 2.491 + | 1.229 + | 3.958 + | + |
| | 2 | 1.170 + | 1.207 + | 2.118 + | 1.914 + | 2.274 + | 1.881 + | 1.282 + | 2.100 + | + |
| | 3 | 2.130 + | 2.032 + | 2.673 + | 2.437 + | 2.432 + | 3.097 + | 1.517 + | 3.958 + | + |
| | 4 | 0.070 | 0.068 | 0.100 | 0.090 | 0.108 | 0.052 | 0.013 | 0.934 + | − |
| | 5 | 0.117 | 0.072 | 0.065 | 0.056 | 0.079 | 0.051 | 0.011 | 0.543 + | − |
| | 6 | 0.053 | 0.058 | 0.145 | 0.054 | 0.135 | 0.079 | 0.013 | 0.487 + | − |
| | 7 | 1.238 + | 1.183 + | 2.277 + | 2.311 + | 2.386 + | 2.138 + | 1.640 + | 1.884 + | + |
| | 8 | 0.091 | 0.069 | 0.185 | 0.158 | 0.161 | 0.049 | 0.009 | 0.689 + | − |
| | 9 | 0.034 | 0.043 | 0.059 | 0.095 | 0.109 | 0.045 | 0.010 | 0.278 | |
| | 10 | 0.968 + | 1.086 + | 2.070 + | 2.171 + | 1.984 + | 2.170 + | 1.118 + | 3.837 + | + |
| | 11 | 0.080 | 0.087 | 0.084 | 0.048 | 0.097 | 0.093 | 0.009 | 0.700 + | − |
| | 12 | 0.024 | 0.064 | 0.032 | 0.053 | 0.067 | 0.053 | 0.009 | 1.124 + | − |
| | 13 | 2.245 + | 2.154 + | 2.404 + | 2.105 + | 2.101 + | 2.503 + | 1.194 + | 3.958 + | + |
| | 14 | 1.177 + | 1.217 + | 0.884 + | 0.748 + | 1.011 + | 2.459 + | 0.250 + | 1.934 + | − |
| | 15 | 0.563 + | 0.552 + | 0.717 + | 0.871 + | 0.853 + | 2.523 + | 0.613 + | 3.958 + | + |
| | 16 | 0.328 + | 0.348 + | 0.077 | 0.075 | 0.372 + | 2.022 + | 0.010 | 0.390 | |
| | 17 | 0.291 + | 0.319 + | 0.082 | 0.128 | 0.418 + | 1.921 + | 0.009 | 2.505 + | + |
| | 18 | 3.388 + | 3.008 + | 1.985 + | 2.158 + | 2.425 + | 2.113 + | 1.228 + | 0.178 | |
| | 19 | 3.176 + | 3.267 + | 1.354 + | 1.045 + | 2.317 + | 1.563 + | 1.415 + | 0.115 | |
| | 20 | 0.090 | 0.075 | 1.380 + | 1.108 + | 1.185 + | 1.024 + | 0.842 + | 0.124 | |
| Normal human serum | 1 | 0.051 | 0.043 | 0.067 | 0.128 | 0.077 | 0.046 | 0.020 | 0.082 | |
| | 2 | 0.065 | 0.067 | 0.029 | 0.064 | 0.074 | 0.059 | 0.021 | 0.095 | |
| | 3 | 0.078 | 0.038 | 0.085 | 0.065 | 0.086 | 0.073 | 0.015 | 0.108 | |
| | 4 | 0.036 | 0.032 | 0.064 | 0.121 | 0.053 | 0.051 | 0.009 | 0.126 | |
| | 5 | 0.042 | 0.044 | 0.113 | 0.057 | 0.047 | 0.087 | 0.011 | 0.115 | |
| | 6 | 0.069 | 0.053 | 0.059 | 0.079 | 0.138 | 0.080 | 0.025 | 0.075 | |
| | 7 | 0.039 | 0.055 | 0.050 | 0.086 | 0.098 | 0.058 | 0.040 | 0.089 | |
| | 8 | 0.036 | 0.061 | 0.067 | 0.094 | 0.061 | 0.064 | 0.018 | 0.121 | |
| | 9 | 0.041 | 0.071 | 0.065 | 0.046 | 0.035 | 0.075 | 0.017 | 0.096 | |
| | 10 | 0.052 | 0.063 | 0.128 | 0.082 | 0.043 | 0.047 | 0.024 | 0.172 | |

Further, the HCV-positive samples were subjected to the inhibition test using the peptides of Sequence 1a7 and Sequence 2. 10 micrograms each of the peptides of Sequence 1a7 and Sequence 2, which were the same peptides as those used to make a solid phase, were added to each sample dissolved in a diluent solution (200 microliters) and then changes in reaction with reagent 1a7 and reagent 2 were observed. As results shown in Table 2, reaction of the HCV-antibody-positive samples were completely inhibited by the addition of 10 micrograms of the peptide of Sequence 1a7, which confirmed high specificity of the reagent. Complete inhibition was observed also with the peptide of Sequence 2, which so far indicated high specificity of the reagent.

| Sample | P1a7 | | | | P2 | | | |
|---|---|---|---|---|---|---|---|---|
| | Peptide of Sequence 1a7 not added | | Peptide of Sequence 1a7 added | | Peptide of Sequence 2 not added | | Peptide of Sequence 2 added | |
| | OD | Judgment | OD | | OD | Judgment | OD | |
| 1 | 1.237 | + | 0.110 | | 1.928 | + | 0.036 | |
| 2 | 1.170 | + | 0.066 | | 2.118 | + | 0.053 | |
| 3 | 2.130 | + | 0.065 | | 2.673 | + | 0.054 | |
| 7 | 1.238 | + | 0.044 | | 2.277 | + | 0.024 | |
| 10 | 0.968 | + | 0.078 | | 2.070 | + | 0.053 | |
| 13 | 2.245 | + | 0.060 | | 2.404 | + | 0.064 | |
| 14 | 1.177 | + | 0.075 | | 0.884 | + | 0.027 | |
| 15 | 0.563 | + | 0.053 | | 0.717 | + | 0.031 | |
| 16 | 0.328 | + | 0.068 | | 0.077 | − | 0.033 | |
| 17 | 0.291 | + | 0.069 | | 0.082 | − | 0.067 | |
| 18 | 3.388 | + | 0.113 | | 1.985 | + | 0.084 | |
| 19 | 3.176 | + | 0.135 | | 1.354 | + | 0.081 | |
| 20 | 0.090 | − | 0.085 | | 1.380 | + | 0.135 | |

[4] Preparation of reagents - 2

Various cross-linking agents were introduced into the peptides synthesized in section [1] and the resultant peptides were used as antigens for reagents.

(1) Introduction of various cross-linking agents into synthetic peptides

A. Introduction of Sulfo-SMCC into synthetic peptides

0.132 mg of Sulfo-SMCC (a product of Pierce) and 0.5 mg of the synthetic peptide (Sequence 1a7 or 2) were dissolved in a 100 mM phosphate buffer solution (pH 7.5) and the resulting solution was mixed. After adjusting the volume of the solution to 0.1 ml, the reaction was allowed to proceed at 25°C for 2 hours and then terminated by adding 10 microliters of 1 M glycine.

B. Introduction of EMCS into synthesized peptides

0.1 mg of EMCS (a product of Dojin Laboratories) was dissolved in 10 microliters of dimethylformamide. The solution was mixed together with 0.5 mg of the synthesized peptide (Sequence 1a7 or 2) dissolved in a 100 mM phosphate buffer solution (pH 7.5). After adjusting the volume of the solution to 0.1 ml, the reaction was allowed to proceed at 25°C for 2 hours and then terminated by adding 10 microliters of 1 M glycine.

C. Introduction of Sulfo-LC-SPDP into synthesized peptides

0.15 mg of Sulfo-LC-SPDP (a product of Pierce) and 0.5 mg of the synthesized peptide (Sequence 1a7 or 2) were dissolved in a 100 mM phosphate buffer solution (pH 7.5) and then the resulting solution was mixed. After adjusting the volume of the solution to 0.1 ml, the reaction was allowed to proceed at 25°C for 2 hours and then terminated by the addition of 10 microliters of 1 M glycine.

D. Introduction of DSS into synthesized peptides

0.2 mg of DSS (a product of Pierce) was dissolved in 50 microliters of dimethylformamide. The solution was added together with 0.5 mg of the synthesized peptide (Sequence 1a7 or 2) to 0.15 ml of a 100 mM phosphate buffer solution (pH 7.5). The reaction was allowed to proceed at 4°C for 8 hours with stirring and then terminated by adding 10 microliters of 1 M glycine.

(2) Preparation of solid phase antigens

According to the method described in [2], plates with a solid phase antigens in which cross-linking agents were introduced as described in A to D above were prepared.

[5] Detection of HCV antibodies - 2

Measurement of antibodies in serum samples of HCV patients was attempted by ELISA using the reagents prepared in [4] above. The inhibition tests using the antigens obtained in [4] were also carried out.

(1) Measurement of antibodies in the serum sample of HCV patients by ELISA

Antibodies in the serum sample of HCV patients were measured according to the method as described in [3] above using the plates with solid phase antigens which were prepared in [4] (2) above. In this regard, samples which were negative with the use of peptides without cross- linking agents (positive with the use of B-1) were primarily selected in order to evaluate the effects of cross-linking agents.

Results are shown in Table 3 and Table 4. Antigenicity of each synthesized peptide was enhanced by the introduction of cross-linking agents; however, the enhancing effect was different depending on the kind of the cross-linking agents used. Sulfo-SMCC, EMCS and Sulfo-LC- SPDP were equally effective whereas DSS was less effective than these three agents. In particular, HCV positive samples were detected without fail with the use of the peptide of Sequence 1a7 with the introduced Sulfo-SMCC. On the other hand, in the case of the peptide of Sequence 2, the failure in detection could not always be eliminated even when cross-linking agents were bound to the peptide. In general, the enhancing effect by cross-linking agents was more prominent in the case of Sequence 1a7. Furthermore, some serum samples which were judged to be negative using B-1 reacted with the antigens in which cross-linking agents had been introduced.

Table 3

In the case of synthesized peptides of Sequence 1a7

| Sample | Peptide with introduced cross-linking agent Peptide | | | | | | | | OD | Judgment | B-1 Judgment |
|---|---|---|---|---|---|---|---|---|---|---|---|
| | Sulfo-SMCC | | EMCS | | Sulfo-LC- | | DSS | | | | |
| | OD | Judgment | OD | Judgment | OD | Judgment | OD | Judgment | OD | Judgment | Judgment |
| A | 0.362 | + | 0.199 | − | 0.139 | − | 0.433 | + | 0.026 | − | + |
| B | 1.038 | + | 1.598 | + | 1.287 | + | 1.117 | + | 0.094 | − | + |
| C | 0.749 | + | 0.759 | + | 0.242 | − | 0.761 | + | 0.038 | − | + |
| D | 0.726 | + | 0.773 | + | 0.412 | + | 0.480 | + | 0.100 | − | + |
| E | 0.786 | + | 0.604 | + | 0.397 | + | 0.955 | + | 0.167 | − | + |
| F | 0.369 | + | 0.264 | + | 0.191 | − | 0.264 | + | 0.060 | − | + |
| G | 1.600 | + | 1.752 | + | 1.105 | + | 0.943 | + | 0.781 | + | + |
| H | 1.412 | + | 1.699 | + | 0.949 | + | 0.893 | + | 0.841 | + | + |
| I | 1.116 | + | 1.300 | + | 1.249 | + | 1.080 | + | 1.370 | + | + |
| J | 1.570 | + | 1.991 | + | 1.315 | + | 1.042 | + | 1.104 | + | + |
| K | 1.399 | + | 1.380 | + | 1.251 | + | 1.065 | + | 1.952 | + | + |
| L | 0.432 | + | ---- | | --- | | --- | | 0.032 | − | − |
| M | 0.126 | − | ---- | | --- | | --- | | 0.091 | − | − |

Table 4

In the case of synthesized peptides of Sequence 2

| Sample | Peptide with introduced cross-linking agent Peptide | | | | | | | | OD | Judgment | B-1 Judgment |
|---|---|---|---|---|---|---|---|---|---|---|---|
| | Sulfo-SMCC | | EMCS | | Sulfo-LC- | | DSS | | | | |
| | OD | Judgment | OD | Judgment | OD | Judgment | OD | Judgment | OD | Judgment | Judgment |
| A | 0.236 | − | 0.250 | − | 0.223 | − | 0.177 | − | 0.380 | − | + |
| B | 1.223 | + | 1.004 | + | 0.893 | + | 0.309 | − | 0.366 | − | + |
| C | 0.264 | − | 0.269 | − | 0.264 | − | 0.167 | − | 0.314 | − | + |
| D | 2.681 | + | 2.702 | + | 2.583 | + | 0.248 | − | 0.556 | + | + |
| E | 2.241 | + | 2.124 | + | 2.161 | + | 0.267 | − | 0.914 | + | + |
| F | 1.872 | + | 1.855 | + | 1.764 | + | 0.807 | + | 0.933 | + | + |
| G | 1.958 | + | 2.041 | + | 1.774 | + | 0.464 | − | 0.993 | + | + |
| H | 2.408 | + | 2.389 | + | 2.236 | + | 0.534 | + | 1.924 | + | + |
| I | 0.881 | + | 0.895 | + | 0.724 | + | 0.222 | − | 0.754 | + | + |
| J | 1.244 | + | 1.426 | + | 1.259 | + | 0.254 | − | 1.025 | + | + |
| K | 2.817 | + | 2.920 | + | 2.829 | + | 0.366 | − | 1.392 | + | + |
| L | 0.224 | − | ---- | | --- | | --- | | 0.099 | − | − |
| M | 0.541 | + | --- | | --- | | --- | | 0.145 | − | − |

(2) Inhibition test

In measuring antibodies using the synthesized peptide (Sequence 1a7) in which Sulfo-SMCC was introduced, changes in reaction were observed by adding 10 micrograms each of the synthesized peptide, the synthesized peptide with introduced Sulfo-SMCC, Sulfo-SMCC, BSA (a product of Sigma) or B-1 to each sample dissolved in a diluent solution (200 microliters). Results are shown in Table 5.

The results indicated that as in same cases such as sample N, the reaction was inhibited by the addition of B-1 and the synthesized peptide with introduced Sulfo-SMCC but not by the synthesized peptide itself or Sulfo-SMCC alone. These results confirmed that the introduction of the cross-linking agents apparently changed the reactivity of the synthesized peptide and thus the synthesized peptides with the cross-linking agents were closely analogous to the antigen which covers all areas of the core region.

Table 5

| Sample | Solid phase antigen: Sequence 1a7 + Sulfo-SMCC | | | | | |
|---|---|---|---|---|---|---|
| | Inhibition Test | | | | | |
| | (-) | Sequence 1a7 | Sequence 1a7 + Sulfo-SMCC | Sulfo-SMCC | BSA | B-1 |
| N | 2.646 | 2.418 | 0.272 | 2.949 | 2.218 | 0.043 |
| O | 0.886 | 0.165 | 0.121 | 0.665 | 0.801 | 0.124 |
| P | 1.102 | 0.262 | 0.251 | 1.180 | 1.221 | 0.210 |
| Q | 0.495 | 0.294 | 0.125 | 0.421 | 0.401 | 0.077 |

[6] Preparation of reagents - 3

Reagents were prepared by additionally binding vehicle proteins to the peptides in which various cross-linking agents were introduced in [4].

(1) Binding of peptide antigens with introduced Sulfo-SMCC cross-linking agent to vehicle proteins

10 mg of BSA (a product of Sigma) or HSA (a product of Sigma) were dissolved in 2 ml of a 100 mM EDTA solution containing 6 M urea. 0.2 ml of n-butanol and 20 mg of $NaBH_4$ were added to the resultant solution. After stirring at 25°C for 30 minutes, 1 ml of 100 mM $NaH_2PO_4$ and 0.4 ml of acetone were added drop-wise to prepare a reduced BSA solution or a reduced HSA solution.

0.1 ml of the reaction solution taken before terminating the reaction with glycine in [4] (the peptide antigen in which the Sulfo-SMCC cross-linking agent was introduced; see [4] (1) A) was added to 0.3 ml each of the solutions thus prepared. The resultant solution was allowed to react at 4°C for 8 hours with stirring and then the reaction was terminated by adding 50 microliters of 50 mM N- ethylmaleimide. Further, purification was carried out using Sephadex G-50 (a product of Pharmacia) to obtain a synthesized peptide with the introduced Sulfo-SMCC cross- linking agent which was bound to the vehicle protein (hereinafter referred to as a BSA-binding peptide and a HSA- binding peptide).

(2) Preparation of solid phase antigen

Solid-phase plates were prepared using the BSA-binding peptide and the HSA-binding peptide according to the method in [2].

[7] Detection of HCV antibodies - 3

According to the method of [2], the solid phase plates prepared in [6] were used to measure antibodies in serum samples of HCV patient (54 specimens) which were judged to be HCV positive by antibody measurement using B-1. Results are shown in Table 6. As shown in Table 6, the BSA-binding peptide antigen and the HSA-binding peptide antigen exhibited equivalent detectability.

Table 6

| Peptide | BSA-binding antigen | | HSA-binding antigen | |
|---|---|---|---|---|
| | Positive/Total | Rate,% | Positive/Total | Rate,% |
| Sequence 1a7 | 53/54 | 98.1 | 53/54 | 98.1 |
| Sequence 2 | 51/54 | 94.4 | 51/54 | 94.4 |

Further, the measurement was also made with normal serum samples. As shown in results in Table 7, non-specific positive reactions were observed for about 2 % of the samples when the BSA-binding peptide antigen was used; in contrast, non-specific positive reactions were negligible when the HSA- binding peptide antigen was used.

Table 7

| Peptide | BSA-binding antigen | | HSA-binding antigen | |
|---|---|---|---|---|
| | Positive/Total | Rate,% | Positive/Total | Rate,% |
| Sequence 1a7 | 7/259 | 2.7 | 1/259 | 0.39 |
| Sequence 2 | 7/259 | 2.7 | 1/259 | 0.39 |

Further, BSA- and HSA-binding reagents were prepared in the same manner using the known peptides (B-1 and N-14) and the same samples were analyzed to compare the results.

[8] Detection of HCV antibodies - 4

The following antigens (HSA-binding antigens) were prepared according to the methods described in [4] and [6] from the peptides of Sequences 1b9 and 1c9 synthesized as described in [1]. Each well of microtiter plates was coated with these peptides according to the method described in [2] (the antigen concentration was 0.5 micrograms/well). Serum samples (105 specimens) from patients who were suspected to be infected with HCV were tested for HCV antibodies using these microtiter plates (using ELISA). Results are shown in Table 8 and are summarized in Table 9.

It was confirmed that the two peptides had almost the same reactivity although they were slightly different in their mode of reactivity.

Sequence 1b9 - spacer (Sulfo-SMCC) - HSA : (referred to as 1b9-HSA)

Sequence 1c9 - spacer (Sulfo-SMCC) - HSA : (referred to as 1c9 - HSA).

Table 8

| Sample No. | 1b9-HSA | 1c9-HSA | | 1b9-HSA | 1c9-HSA |
|---|---|---|---|---|---|
| 1 | 1.065* | 1.022* | 54 | 0.046 | 0.038 |
| 2 | 1.118* | 0.980* | 55 | 0.030 | 0.025 |
| 3 | 2.558* | 2.551* | 56 | 0.187 | 0.345* |
| 4 | 0.070 | 0.045 | 57 | 0.662* | 0.270* |
| 5 | 0.199 | 0.122 | 58 | 1.727* | 1.486* |
| 6 | 0.141 | 0.646* | 59 | 0.065 | 0.049 |
| 7 | 2.400* | 2.094* | 60 | 1.732* | 1.920* |
| 8 | 0.006 | 0.002 | 61 | 0.064 | 0.067 |
| 9 | 0.077 | 0.059 | 62 | 0.217 | 0.125 |
| 10 | 1.724* | 1.963* | 63 | 0.127 | 0.233* |
| 11 | 0.068 | 0.037 | 64 | 2.784* | 2.711* |
| 12 | 0.037 | 0.058 | 65 | 0.035 | 0.027 |
| 13 | 2.607* | 2.422 | 66 | 0.424* | 0.247* |
| 14 | 2.510* | 1.865* | 67 | 0.031 | 0.022 |
| 15 | 0.604* | 0.370* | 68 | 2.340* | 1.050* |
| 16 | 0.370* | 0.308* | 69 | 1.450* | 0.972* |
| 17 | 0.190 | 0.159 | 70 | 0.500* | 0.171 |
| 18 | 0.489* | 0.406* | 71 | 0.390* | 0.181 |
| 19 | 0.054 | 0.039 | 72 | 0.042 | 0.031 |
| 20 | 0.140 | 0.062 | 73 | 0.637* | 0.164 |
| 21 | 0.237 | 0.137 | 74 | 2.402* | 2.347* |
| 22 | 0.168 | 0.125 | 75 | 2.043* | 1.958* |
| 23 | 0.084 | 0.055 | 76 | 0.567* | 0.629* |
| 24 | 0.072 | 0.029 | 77 | 0.419* | 0.301* |
| 25 | 2.566* | 2.374* | 78 | 1.733* | 1.488* |
| 26 | 0.444* | 0.065 | 79 | 1.267* | 0.949* |
| 27 | 1.059* | 0.505* | 80 | 0.083 | 0.056 |
| 28 | 0.080 | 0.051 | 81 | 0.033 | 0.033 |
| 29 | 0.052 | 0.035 | 82 | 2.629* | 2.357* |
| 30 | 0.083 | 0.028 | 83 | 2.026* | 2.091* |
| 31 | 0.064 | 0.049 | 84 | 0.364* | 2.700* |
| 32 | 0.078 | 0.070 | 85 | 2.763* | 2.750* |
| 33 | 0.437* | 0.644* | 86 | 1.855* | 1.650* |
| 34 | 0.718* | 0.071 | 87 | 0.126 | 0.113 |
| 35 | 2.682* | 2.614* | 88 | 0.081 | 0.074 |
| 36 | 0.273 | 0.227* | 89 | 0.032 | 0.041 |
| 37 | 0.034 | 0.031 | 90 | 0.038 | 0.032 |
| 38 | 0.190 | 0.135 | 91 | 2.122* | 1.611* |
| 39 | 0.639* | 0.072 | 92 | 2.063* | 1.840* |
| 40 | 0.893* | 0.840* | 93 | 0.381* | 0.344* |
| 41 | 0.368* | 0.117 | 94 | 0.092 | 0.061 |
| 42 | 2.672* | 2.266* | 95 | 0.811* | 1.516* |
| 43 | 0.233 | 0.164 | 96 | 0.015 | 0.101 |
| 44 | 0.041 | 0.033 | 97 | 1.938* | 2.103* |
| 45 | 0.084 | 0.063 | 98 | 0.971* | 0.124 |
| 46 | 0.600* | 0.450* | 99 | 0.067 | 0.055 |
| 47 | 2.035* | 2.178* | 100 | 0.062 | 0.059 |
| 48 | 0.420* | 0.322* | 101 | 0.585* | 0.250* |
| 49 | 0.254 | 0.210* | 102 | 0.026 | 0.023 |
| 50 | 0.040 | 0.030 | 103 | 1.935* | 2.113* |
| 51 | 0.035 | 0.028 | 104 | 0.042 | 0.038 |
| 52 | 0.034 | 0.033 | 105 | 0.115 | 0.103 |
| 53 | 0.068 | 0.054 | | | |

Table 9

Samples suspected to be hepatitis C positive (105 specimens) (comparison between the reagents of the present invention)

| P 1 c 9 | P 1 b 9 | | |
|---|---|---|---|
| | + ( % ) | − ( % ) | Total |
| + (%) | 4 3 (40. 9) | 5 ( 4. 8) | 4 8 (45. 7) |
| − (%) | 9 ( 8. 6) | 4 8 (45. 7) | 5 7 (54. 3) |
| Total | 5 2 (49. 5) | 5 3 (50. 5) | 1 0 5 (100) |

Samples suspected to be hepatitis C positive (105 specimens) (comparison between the reagents of the present invention)

[9] Detection of HCV antibodies - 5

The peptides of Sequence 1b9 and Sequence 3 were used to prepare antigens which were separately bound to HSA via a spacer (Sulfo-SMCC). These antigens were then combined at a 1:1 ratio to prepare a reagent (referred to as P1b9*3). Solid phase microtiter plates were prepared using the resultant reagent according to the method described in [2] (at a concentration of 0.5 + 0.5 = 1.0 microgram/well) and used for the detection of HCV antibodies in various samples (by the ELISA method, partly by the RIA method). Furthermore, for comparative purposes, the same samples were analyzed in the same manner using the reagents prepared from the known peptides (B-1 and N-14).

Samples used were B-1 positive samples (139 specimens), RF-positive samples (50 specimens), samples from normal subjects (100 specimens), as well as the above- mentioned samples which were suspected to be HCV positive.

The RF-positive samples were used because RF (rheumatoid factor) is known to be responsible for HCV false positive reactions and thus samples which are positive due to RF interference may tend to induce non-specific reactions.

Results are shown in Table 10 (for the samples which were suspected to be infected with HCV), Table 13 (for the B- 1 positive samples), Table 16 (for the RF-positive samples) and Table 19 (for the normal samples). Further, summarized results for each of the samples are shown in Tables 11 and 12 (corresponding to Table 10), Tables 14 and 15 (corresponding to Table 13), Tables 17 and 18 (corresponding to Table 16) and Table 20 (corresponding to Table 19). As to the samples which were suspected to be infected with HCV, some samples which were negative with N-14 reagent exhibited positive reaction with P1b9*3; detectability with P1b9*3 was higher than that with B-1 (Tables 10, 11 and 12). As clearly shown in Tables 13, 14 and 15 in which reagents P1b9*3 and N-14 were compared using the B-1 positive samples as the standard samples, detectability was higher with P1b9*3 reagent than with N-14 reagent. As to the RF-positive samples, B-1 reagent was highly sensitive and thus tended to be susceptible to RF interference whereas P1b9*3 reagent was as sensitive as N-14 reagent and less susceptible to RF interference (Table 16, 17 and 18). As to the normal samples, none of the reagents P1b9*3, B-1 or N-14 exhibited positive reactions; however, reagent B-1 had a tendency to overmeasure positive reactions (showing relatively high optical density), whereas P1b9*3 reagent showed such a tendency to a lesser extent and was almost the same as N-14 reagent (Table 19). Further, cutoff values calculated from the results were 0.319, 0.445 and 0.037 for reagents P1b9*3, B-1 and N-14, respectively.

Thus, P1b9*3 reagent in which the peptides of the present invention were combined proved to be superior to N-14 reagent and equivalent or superior to B-1 reagent with respect to HCV antibody detectability or specificity.

Table 10

| Sample No. | Solid-phase antigen P1b9*3 | B-1 | N-14 | Sample No. | Solid-phase antigen P1b9*3 | B-1 | N-14 |
|---|---|---|---|---|---|---|---|
| 1 | 2.553 * | 2.491 * | 1.229 * | 51 | 0.099 | 0.058 | 0.010 |
| 2 | 1.926 * | 1.881 * | 1.282 * | 52 | 0.073 | 0.064 | 0.023 |
| 3 | 3.110 * | 3.097 * | 1.517 * | 53 | 0.090 | 0.075 | 0.010 |
| 4 | 0.102 | 0.052 | 0.013 | 54 | 0.056 | 0.047 | 0.009 |
| 5 | 0.200 | 0.051 | 0.011 | 55 | 0.058 | 0.051 | 0.009 |
| 6 | 0.274 | 0.079 | 0.013 | 56 | 0.125 | 0.050 | 0.009 |
| 7 | 3.025 * | 2.138 * | 1.640 * | 57 | 2.139 * | 2.359 * | 0.915 * |
| 8 | 0.293 | 0.049 | 0.009 | 58 | 2.799 * | 2.300 * | 1.210 * |
| 9 | 0.062 | 0.045 | 0.010 | 59 | 0.083 | 0.060 | 0.009 |
| 10 | 2.438 * | 2.170 * | 1.118 * | 60 | 2.645 * | 2.408 * | 0.821 * |
| 11 | 0.070 | 0.093 | 0.009 | 61 | 0.088 | 0.053 | 0.009 |
| 12 | 0.060 | 0.053 | 0.009 | 62 | 2.135 * | 2.349 * | 1.164 * |
| 13 | 2.937 * | 2.503 * | 1.194 * | 63 | 0.113 | 0.051 | 0.009 |
| 14 | 2.737 * | 2.459 * | 0.250 * | 64 | 3.077 * | 3.306 * | 1.304 * |
| 15 | 1.067 * | 2.523 * | 0.613 * | 65 | 0.058 | 0.051 | 0.009 |
| 16 | 2.744 * | 2.127 * | 1.253 * | 66 | 2.812 * | 2.411 * | 1.484 * |
| 17 | 0.082 | 0.098 | 0.028 | 67 | 0.065 | 0.048 | 0.009 |
| 18 | 2.537 * | 1.826 * | 1.030 * | 68 | 2.832 * | 2.116 * | 0.838 * |
| 19 | 0.061 | 0.055 | 0.009 | 69 | 3.068 * | 3.032 * | 1.394 * |
| 20 | 0.155 | 0.054 | 0.010 | 70 | 0.422 * | 0.063 | 0.009 |
| 21 | 0.148 | 0.080 | 0.167 | 71 | 2.877 * | 2.800 * | 1.234 * |
| 22 | 0.090 | 0.161 | 0.030 | 72 | 0.052 | 0.051 | 0.009 |
| 23 | 0.119 | 0.173 | 0.010 | 73 | 1.010 * | 1.335 * | 0.102 |
| 24 | 0.082 | 0.054 | 0.020 | 74 | 2.747 * | 2.248 * | 0.471 * |
| 25 | 3.046 * | 3.252 * | 1.261 * | 75 | 3.016 * | 3.066 * | 1.619 * |
| 26 | 0.326 * | 2.529 * | 0.010 | 76 | 1.469 * | 2.416 * | 0.815 * |
| 27 | 1.328 * | 2.228 * | 0.237 * | 77 | 1.708 * | 2.415 * | 0.889 * |
| 28 | 0.085 | 0.050 | 0.010 | 78 | 2.394 * | 2.058 * | 0.011 |
| 29 | 0.101 | 0.207 | 0.072 | 79 | 1.838 * | 2.291 * | 0.348 * |
| 30 | 0.071 | 0.047 | 0.008 | 80 | 0.112 | 0.052 | 0.009 |
| 31 | 0.085 | 0.062 | 0.009 | 81 | 0.056 | 0.050 | 0.008 |
| 32 | 0.066 | 0.054 | 0.009 | 82 | 3.135 * | 2.510 * | 1.474 * |
| 33 | 0.456 * | 2.022 * | 0.010 | 83 | 2.776 * | 2.833 * | 1.018 * |
| 34 | 0.826 * | 0.050 | 0.009 | 84 | 1.205 * | 2.567 * | 0.472 * |
| 35 | 3.187 * | 2.833 * | 1.288 * | 85 | 3.144 * | 1.981 * | 1.590 * |
| 36 | 0.374 * | 1.921 * | 0.009 | 86 | 2.870 * | 1.855 * | 1.540 * |
| 37 | 0.068 | 0.046 | 0.009 | 87 | 0.632 * | 1.175 * | 0.175 |
| 38 | 0.222 | 0.059 | 0.011 | 88 | 0.081 | 0.047 | 0.010 |
| 39 | 2.390 * | 2.095 * | 1.363 * | 89 | 0.069 | 0.050 | 0.010 |
| 40 | 1.351 * | 2.366 * | 0.009 | 90 | 0.071 | 0.045 | 0.010 |
| 41 | 1.719 * | 0.073 | 0.200 | 91 | 2.198 * | 1.855 * | 0.773 * |
| 42 | 3.055 * | 2.762 * | 0.834 * | 92 | 2.912 * | 1.726 * | 1.276 * |
| 43 | 0.132 | 0.051 | 0.009 | 93 | 2.243 * | 1.227 * | 0.686 * |
| 44 | 0.050 | 0.100 | 0.009 | 94 | 0.226 | 0.057 | 0.009 |
| 45 | 0.104 | 0.087 | 0.009 | 95 | 1.579 * | 0.936 * | 0.035 |
| 46 | 2.822 * | 2.436 * | 1.427 * | 96 | 0.127 | 0.052 | 0.010 |
| 47 | 2.575 * | 2.320 * | 1.099 * | 97 | 2.812 * | 2.428 * | 1.384 * |
| 48 | 0.234 | 0.087 | 0.009 | 98 | 2.589 * | 2.304 * | 1.329 * |
| 49 | 2.100 * | 1.696 * | 0.838 * | 99 | 0.119 | 0.053 | 0.010 |
| 50 | 0.051 | 0.080 | 0.009 | 100 | 0.150 | 0.061 | 0.010 |
| 104 | 1.088 * | 2.152 * | (539) | 112 | 0.069 | 0.074 | (446) |
| 145 | 2.671 * | 1.889 * | (1253) | 180 | 0.117 | 0.793 * | (346) |
| 194 | 0.428 * | 2.092 * | (796) | | | | ( )RIAcpm |

17

Table 11

Samples suspected to be hepatitis C positive
(105 specimens)
(comparison between the reagent of the present
invention and B-1)

| B - 1 | | | |
|---|---|---|---|
| P 1 b 9 ∗ 3 | + ( % ) | − ( % ) | Total |
| + (%) | 5 3 (50. 5) | 3 ( 2. 9) | 5 6 (53. 3) |
| − (%) | 1 ( 1. 0) | 4 8 (45. 7) | 4 9 (46. 7) |
| Total | 5 4 (51. 4) | 5 1 (48. 6) | 1 0 5 (100) |

Table 12

Samples suspected to be hepatitis C positive
(105 specimens)
(Comparison between the reagent of the present
invention and N-14)

| N - 1 4 | | | |
|---|---|---|---|
| P 1 b 9 ∗ 3 | + ( % ) | − ( % ) | Total |
| + (%) | 4 2 (40. 0) | 1 4 (13. 3) | 5 6 (53. 3) |
| − (%) | 0 ( 0. 0) | 4 9 (46. 7) | 4 9 (46. 7) |
| Total | 4 2 (40. 0) | 6 3 (60. 0) | 1 0 5 (100) |

Table 13

B-1-positive samples
(139 specimens)

| | Solid-phase antigen | | |
|---|---|---|---|
| | P1b9*3 | B-1 | N-14 |
| 1 | 2.500 * | 3.377 * | 0.762 * |
| 2 | 1.183 * | 2.870 * | 0.040 |
| 3 | 0.442 * | 2.645 * | 0.049 |
| 4 | 2.390 * | 3.179 * | 1.518 * |
| 5 | 3.029 * | 3.324 * | 1.407 * |
| 6 | 0.223 * | 3.990 * | 0.117 |
| 7 | 2.709 * | 3.699 * | 0.041 |
| 8 | 3.102 * | 3.173 * | 1.261 * |
| 9 | 3.067 * | 3.330 * | 1.342 * |
| 10 | 2.983 * | 2.980 * | 1.494 * |
| 11 | 2.910 * | 3.593 * | |
| 12 | 0.307 * | 2.987 * | 0.156 |
| 13 | 1.033 * | 3.037 * | 0.806 * |
| 14 | 2.694 * | 3.329 * | 0.830 * |
| 15 | 2.938 * | 3.604 * | 0.954 * |
| 16 | 2.613 * | 2.869 * | 1.582 * |
| 17 | 2.301 * | 3.038 * | 1.039 * |
| 18 | 1.930 * | 3.344 * | 1.235 * |
| 19 | 1.803 * | 2.581 * | 0.449 * |
| 20 | 2.754 * | 3.625 * | 1.365 * |
| 21 | 2.587 * | 3.064 * | 1.478 * |
| 22 | 1.956 * | 3.477 * | 0.570 * |
| 23 | 2.139 * | 3.168 * | 1.487 * |
| 24 | 3.008 * | 3.990 * | 1.280 * |
| 25 | 3.049 * | 3.496 * | 1.836 * |
| 26 | 2.875 * | 3.990 * | 1.685 * |
| 27 | 1.155 * | 2.565 * | 0.765 * |
| 28 | 2.350 * | 3.990 * | 1.376 * |
| 29 | 0.348 * | 3.026 * | 0.333 * |
| 30 | 1.987 * | 3.990 * | 0.300 * |
| 31 | 2.494 * | 2.691 * | 1.140 * |
| 32 | 1.438 * | 2.903 * | 0.990 * |
| 33 | 1.408 * | 3.000 * | 0.113 |
| 34 | 2.765 * | 2.705 * | 1.720 * |
| 35 | 1.105 * | 2.840 * | 1.048 * |
| 36 | 1.695 * | 2.873 * | 1.219 * |
| 37 | 2.837 * | 2.712 * | 1.482 * |
| 38 | 1.828 * | 2.706 * | |
| 39 | 0.364 * | 3.204 * | 1.620 * |
| 40 | 1.273 * | 2.607 * | 0.043 |
| 41 | 1.862 * | 2.478 * | 1.341 * |
| 42 | 1.736 * | 2.916 * | 1.215 * |
| 43 | 0.963 * | 2.475 * | 0.032 |
| 44 | 2.875 * | 2.822 * | 1.218 * |
| 45 | 0.030 | 1.371 * | 0.029 |
| 46 | 2.728 * | 2.979 * | 1.914 * |
| 47 | 0.074 | 2.548 * | |
| 48 | 0.169 * | 2.836 * | |
| 49 | 2.977 * | 2.890 * | 0.150 |
| 50 | 2.764 * | 3.165 * | 1.679 * |

19

Table 13 (continued)

| | Solid-phase antigen | | |
|---|---|---|---|
| | P1b9*3 | B-1 | N-14 |
| 51 | 0.742 * | 2.442 * | 1.014 * |
| 52 | 0.595 * | 3.128 * | 1.336 * |
| 53 | 2.804 * | 2.622 * | 1.570 * |
| 54 | 1.705 * | 2.662 * | 1.355 * |
| 55 | 0.065 | 1.620 * | |
| 56 | 1.624 * | 2.781 * | 0.778 * |
| 57 | 0.042 | 2.634 * | |
| 58 | 0.231 * | 2.440 * | 0.028 |
| 59 | 2.354 * | 2.271 * | 1.730 * |
| 60 | 3.259 * | 2.299 * | 0.190 |
| 61 | 2.161 * | 2.283 * | 1.120 * |
| 62 | 1.725 * | 2.085 * | 1.663 * |
| 63 | 1.957 * | 2.201 * | 1.786 * |
| 64 | 0.997 * | 1.998 * | 1.124 * |
| 65 | 2.876 * | 2.363 * | 1.526 * |
| 66 | 2.989 * | 2.537 * | 1.815 * |
| 67 | 2.883 * | 2.745 * | 1.783 * |
| 68 | 2.414 * | 2.482 * | 1.388 * |
| 69 | 2.309 * | 2.920 * | 1.126 * |
| 70 | 0.301 * | 2.130 * | 0.144 |
| 71 | 1.305 * | 2.458 * | 0.670 * |
| 72 | 2.594 * | 2.811 * | 0.726 * |
| 73 | 2.009 * | 2.512 * | 1.087 * |
| 74 | 0.225 * | 2.541 * | |
| 75 | 0.842 * | 2.327 * | 0.844 * |
| 76 | 1.296 * | 2.529 * | 0.472 * |
| 77 | 0.149 * | 1.758 * | |
| 78 | 2.683 * | 2.218 * | 2.179 * |
| 79 | 1.076 * | 2.438 * | 0.090 |
| 80 | 3.018 * | 2.146 * | 1.696 * |
| 81 | 0.511 * | 2.586 * | 0.167 |
| 82 | 1.354 * | 2.338 * | 1.245 * |
| 83 | 0.557 * | 2.008 * | 0.052 |
| 84 | 3.068 * | 2.583 * | 1.804 * |
| 85 | 3.120 * | 2.420 * | 1.577 * |
| 86 | 3.290 * | 3.423 * | 1.622 * |
| 87 | 3.043 * | 3.418 * | 1.441 * |
| 88 | 1.665 * | 2.732 * | 0.951 * |
| 89 | 0.159 * | 2.896 * | 0.053 |
| 90 | 2.766 * | 3.146 * | 1.666 * |
| 91 | 2.438 * | 3.528 * | 0.138 |
| 92 | 2.791 * | 2.754 * | 1.687 * |
| 93 | 0.715 * | 2.713 * | 0.492 * |
| 94 | 3.026 * | 3.376 * | 0.577 * |
| 95 | 0.654 * | 3.397 * | 0.046 |
| 96 | 0.457 * | 1.949 * | 0.081 |
| 97 | 0.143 * | 2.244 * | 0.053 |
| 98 | 1.545 * | 2.416 * | 0.781 * |
| 99 | 0.539 * | 2.029 * | 0.595 * |
| 100 | 2.541 * | 2.199 * | 1.283 * |

Table 13 (continued)

| | Solid-phase antigen | | |
|---|---|---|---|
| | P1b9*3 | B-1 | N-14 |
| 101 | 2.778 * | 2.466 * | 1.310 * |
| 102 | 2.761 * | 2.287 * | 1.875 * |
| 103 | 2.474 * | 2.029 * | |
| 104 | 2.184 * | 2.313 * | 0.541 * |
| 105 | 0.949 * | 1.986 * | 0.987 * |
| 106 | 0.065 | 0.678 * | 0.058 |
| 107 | 1.279 * | 2.084 * | |
| 108 | 2.649 * | 2.334 * | 1.274 * |
| 109 | 2.368 * | 2.314 * | 1.741 * |
| 110 | 1.698 * | 2.168 * | |
| 111 | 1.092 * | 2.962 * | 0.953 * |
| 112 | 2.064 * | 3.027 * | 1.601 * |
| 113 | 2.865 * | 3.368 * | |
| 114 | 0.128 | 3.990 * | 0.031 |
| 115 | 2.963 * | 3.265 * | |
| 116 | 2.852 * | 3.045 * | 1.976 * |
| 117 | 1.173 * | 2.943 * | |
| 118 | 2.809 * | 3.194 * | 1.645 * |
| 119 | 2.152 * | 3.459 * | |
| 120 | 1.930 * | 2.842 * | 1.640 * |
| 121 | 0.716 * | 3.095 * | 0.785 * |
| 122 | 1.729 * | 3.138 * | 0.037 |
| 123 | 2.874 * | 3.990 * | 1.872 * |
| 124 | 0.769 * | 2.973 * | |
| 125 | 2.711 * | 3.697 * | 0.121 |
| 126 | 2.619 * | 2.836 * | 1.871 * |
| 127 | 1.628 * | 2.620 * | 0.027 |
| 128 | 2.758 * | 3.093 * | 1.646 * |
| 129 | 2.613 * | 3.129 * | 0.054 |
| 130 | 0.171 * | 2.142 * | |
| 131 | 2.965 * | 3.259 * | 1.719 * |
| 132 | 3.037 * | 3.482 * | 1.682 * |
| 133 | 2.625 * | 3.670 * | |
| 134 | 2.661 * | 3.035 * | |
| 135 | 1.046 * | 2.811 * | |
| 136 | 2.800 * | 3.990 * | 1.171 * |
| 137 | 2.945 * | 3.658 * | 0.028 |
| 138 | 0.057 | 1.296 * | |
| 139 | 1.680 * | 2.466 * | 1.686 * |
| negative | 7/139 | 0/1 | 28/118 |
| ( | 5.0 | | 23.7 |

Table 14

| B-1-positive samples (139 specimens) (compsrison between the reagent of the present invention and reagent B-1) | |
|---|---|
| P1b9*3 | B-1 + (%) |
| + (%) | 132(95.5) |
| - (%) | 7(5.0) |
| Total | 139(100) |

Table 15

B-1-positive samples (118 out of 139 specimens) (comparison between the reagent of the present invention and reagent N-14)

| P1b9*3 | N－1 4 | | |
|---|---|---|---|
| | ＋ （％） | － （％） | Total |
| ＋ (%) | 9 0 (76. 3) | 2 5 (21. 2) | 1 1 5 (97. 5) |
| － (%) | 0 ( 0. 0) | 3 ( 2. 5) | 3 ( 2. 5) |
| Total | 9 0 (76. 3) | 2 8 (23. 7) | 1 1 8 (100) |

Table 16

RF-positive samples (50 specimens)

| No. | N-14 | B-1 | P1b9*3 | No. | N-14 | B-1 | P1b9*3 |
|---|---|---|---|---|---|---|---|
| 1 | 0.014 | 0.138 | 0.172 | 26 | 0.010 | 0.290 | 0.093 |
| 2 | 0.815* | 3.397* | 2.644* | 27 | 0.012 | 0.045 | 0.074 |
| 3 | 0.014 | 0.057 | 0.924* | 28 | 0.025 | 0.172 | 0.121 |
| 4 | 0.015 | 0.156 | 0.112 | 29 | 0.008 | 0.025 | 0.099 |
| 5 | 0.010 | 0.134 | 0.214 | 30 | 0.008 | 0.142 | 0.263 |
| 6 | 0.006 | 0.058 | 0.045 | 31 | 0.018 | 0.060 | 0.166 |
| 7 | 0.008 | 0.033 | 0.081 | 32 | 0.013 | 0.064 | 0.082 |
| 8 | 0.009 | 0.167 | 0.093 | 33 | 0.013 | 0.117 | 0.122 |
| 9 | 0.015 | 0.187 | 0.160 | 34 | 0.014 | 0.237 | 0.105 |
| 10 | 0.013 | 0.041 | 0.105 | 35 | 0.306* | 2.921* | 0.951* |
| 11 | 0.018 | 0.094 | 0.141 | 36 | 0.009 | 0.121 | 0.086 |
| 12 | 0.007 | 0.075 | 0.048 | 37 | 0.008 | 0.069 | 0.158 |
| 13 | 0.010 | 0.046 | 0.083 | 38 | 0.090* | 0.988* | 0.286 |
| 14 | 0.011 | 0.051 | 0.083 | 39 | 0.017 | 0.046 | 0.146 |
| 15 | 0.037* | 0.173 | 0.156 | 40 | 0.008 | 2.696* | 1.692* |
| 16 | 0.009 | 0.025 | 0.136 | 41 | 0.008 | 0.062 | 0.079 |
| 17 | 0.012 | 0.212 | 0.195 | 42 | 0.009 | 0.067 | 0.055 |
| 18 | 0.011 | 1.671* | 0.488* | 43 | 0.765* | 3.156* | 2.539* |
| 19 | 0.387* | 2.888* | 1.034* | 44 | 0.024 | 0.706* | 0.178 |
| 20 | 0.019 | 0.600* | 0.282 | 45 | 0.008 | 0.652* | 0.119 |
| 21 | 0.013 | 0.099 | 0.149 | 46 | 0.014 | 0.077 | 0.107 |
| 22 | 0.017 | 0.333 | 0.258 | 47 | 0.013 | 0.023 | 0.069 |
| 23 | 0.011 | 0.202 | 0.074 | 48 | 0.011 | 0.051 | 0.091 |
| 24 | 0.009 | 0.042 | 0.137 | 49 | 0.018 | 0.476 | 0.087 |
| 25 | 0.006 | 0.037 | 0.097 | Cut | | | |
| 26 | 0.009 | 0.066 | 0.101 | off | 0.035 | 0.541 | 0.319 |

Table 17

RF-positive samples (50 specimens)
(comparison between the reagent of the present
invention and reagent B-1)

| P1b9*3 | B — 1 | | |
|---|---|---|---|
| | + ( % ) | — ( % ) | Total |
| + (%) | 6 (12. 0) | 1 ( 2. 0) | 7 (14. 0) |
| — (%) | 4 ( 8. 0) | 3 9 (78. 0) | 4 3 (86. 0) |
| Total | 1 0 (20. 0) | 4 0 (80. 0) | 5 0 (100) |

Table 18

RF-positive samples (50 specimens)
(comparison between the reagent of the present
invention and reagent N-14)

| P1b9*3 | N — 1 4 | | |
|---|---|---|---|
| | + ( % ) | — ( % ) | Total |
| + (%) | 4 ( 8. 0) | 3 ( 6. 0) | 7 (14. 0) |
| — (%) | 2 ( 4. 0) | 4 1 (82. 0) | 4 3 (86. 0) |
| Total | 6 (12. 0) | 4 4 (88. 0) | 5 0 (100) |

Table 19

Normal serum samples (100 sepcimens)

| Sample No. | solid-phase antigen | | | Sample No. | solid-phase antigen | | |
|---|---|---|---|---|---|---|---|
| | P1b9*3 | B-1 | N-14 | | P1bP*3 | B-1 | N-14 |
| 101 | 0.165 | 0.495 | 0.016 | 151 | 0.039 | 0.028 | 0.005 |
| 102 | 0.175 | 0.219 | 0.012 | 152 | 0.251 | 0.178 | 0.016 |
| 103 | 0.071 | 0.314 | 0.012 | 153 | 0.210 | 0.078 | 0.018 |
| 104 | 0.066 | 0.123 | 0.010 | 154 | 0.107 | 0.163 | 0.008 |
| 105 | 0.098 | 0.175 | 0.012 | 155 | 0.058 | 0.261 | 0.004 |
| 106 | 0.155 | 0.061 | 0.013 | 156 | 0.106 | 0.141 | 0.018 |
| 107 | 0.248 | 0.065 | 0.007 | 157 | 0.086 | 0.072 | 0.002 |
| 108 | 0.389 | 0.086 | 0.012 | 158 | 0.093 | 0.109 | 0.009 |
| 109 | 0.076 | 0.146 | 0.021 | 159 | 0.052 | 0.024 | 0.023 |
| 110 | 0.102 | 0.170 | 0.020 | 160 | 0.095 | 0.207 | 0.014 |
| 111 | 0.058 | 0.214 | 0.016 | 161 | 0.042 | 0.022 | 0.007 |
| 112 | 0.070 | 0.188 | 0.013 | 162 | 0.051 | 0.039 | 0.007 |
| 113 | 0.125 | 0.066 | 0.013 | 163 | 0.143 | 0.119 | 0.007 |
| 114 | 0.052 | 0.151 | 0.006 | 164 | 0.082 | 0.081 | 0.005 |
| 115 | 0.083 | 0.243 | 0.014 | 165 | 0.080 | 0.057 | 0.016 |
| 116 | 0.068 | 0.067 | 0.007 | 166 | 0.074 | 0.049 | 0.014 |
| 117 | 0.048 | 0.204 | 0.015 | 167 | 0.070 | 0.033 | 0.008 |
| 118 | 0.093 | 0.208 | 0.009 | 168 | 0.058 | 0.088 | 0.006 |
| 119 | 0.038 | 0.053 | 0.006 | 169 | 0.054 | 0.037 | 0.016 |
| 120 | 0.074 | 0.132 | 0.013 | 170 | 0.057 | 0.054 | 0.011 |
| 121 | 0.128 | 0.574 | 0.016 | 171 | 0.059 | 0.145 | 0.011 |
| 122 | 0.068 | 0.033 | 0.008 | 172 | 0.152 | 0.236 | 0.011 |
| 123 | 0.063 | 0.449 | 0.009 | 173 | 0.141 | 0.036 | 0.004 |
| 124 | 0.053 | 0.068 | 0.010 | 174 | 0.053 | 0.134 | 0.006 |
| 125 | 0.093 | 0.124 | 0.014 | 175 | 0.082 | 0.053 | 0.006 |
| 126 | 0.051 | 0.109 | 0.008 | 176 | 0.451 | 0.182 | 0.012 |
| 127 | 0.042 | 0.062 | 0.013 | 177 | 0.244 | 0.090 | 0.005 |
| 128 | 0.049 | 0.127 | 0.013 | 178 | 0.066 | 0.094 | 0.008 |
| 129 | 0.052 | 0.023 | 0.012 | 179 | 0.075 | 0.040 | 0.006 |
| 130 | 0.081 | 0.162 | 0.013 | 180 | 0.090 | 0.059 | 0.005 |
| 131 | 0.070 | 0.161 | 0.032 | 181 | 0.090 | 0.187 | 0.007 |
| 132 | 0.056 | 0.083 | 0.014 | 182 | 0.056 | 0.046 | 0.031 |
| 133 | 0.136 | 0.069 | 0.014 | 183 | 0.051 | 0.067 | 0.007 |
| 134 | 0.077 | 0.311 | 0.027 | 184 | 0.041 | 0.025 | 0.005 |
| 135 | 0.049 | 0.059 | 0.011 | 185 | 0.093 | 0.131 | 0.007 |
| 136 | 0.117 | 0.177 | 0.014 | 186 | 0.085 | 0.078 | 0.005 |
| 137 | 0.050 | 0.064 | 0.008 | 187 | 0.057 | 0.116 | 0.005 |
| 138 | 0.080 | 0.031 | 0.012 | 188 | 0.069 | 0.092 | 0.004 |
| 139 | 0.061 | 0.192 | 0.014 | 189 | 0.067 | 0.056 | 0.012 |
| 140 | 0.084 | 0.042 | 0.017 | 190 | 0.034 | 0.132 | 0.005 |
| 141 | 0.039 | 0.087 | 0.012 | 191 | 0.051 | 0.106 | 0.010 |
| 142 | 0.043 | 0.073 | 0.009 | 192 | 0.162 | 0.096 | 0.014 |
| 143 | 0.083 | 0.107 | 0.008 | 193 | 0.100 | 0.050 | 0.009 |
| 144 | 0.084 | 0.198 | 0.014 | 194 | 0.050 | 0.045 | 0.007 |
| 145 | 0.068 | 0.029 | 0.008 | 195 | 0.055 | 0.050 | 0.007 |
| 146 | 0.069 | 0.067 | 0.010 | 196 | 0.075 | 0.604 | 0.007 |
| 147 | 0.090 | 0.035 | 0.028 | 197 | 0.047 | 0.061 | 0.007 |
| 148 | 0.065 | 0.088 | 0.009 | 198 | 0.159 | 0.084 | 0.011 |
| 149 | 0.054 | 0.085 | 0.005 | 199 | 0.053 | 0.084 | 0.009 |
| 150 | 0.208 | 0.250 | 0.004 | 200 | 0.043 | 0.041 | 0.007 |

POSSIBLE USE IN INDUSTRIAL FIELD

As mentioned above, according to the present invention, it is possible to detect HCV infection accurately and at an early stage.

Table of Sequence

Sequence number: 1

Sequence length: 10

Sequence type: Amino acid

Topology: Linear

Molecule type: Peptide

Other information: A peptide which is reactive to antibodies against the HCV core region

Sequence description:

Arg Leu Gly Pro Arg Leu Gly Arg Arg Pro
  1                   5                        10

Sequence number: 2

Length of sequence: 10

Type of sequence: Amino acid

Topology: Normal chain

Kind of sequence: Peptide

Other information: A peptide which is reactive to antibodies against the HCV core region

Sequence:

Arg Lys Gly Pro Arg Leu Gly Arg Arg Pro
  1                   5                        10

Sequence number: 3

Length of sequence: 10

Type of sequence: Amino acid

Topology: Normal chain

Kind of sequence: Peptide

Other information: A peptide which is reactive to anti-bodies against the HCV core region

Sequence:

Arg Arg Gly Pro Arg Leu Gly Arg Arg Pro

1       5       10

Sequence number: 4

Length of sequence: 11

Type of sequence: Amino acid

Topology: Normal chain

Kind of sequence: Peptide

Other information: A peptide which is reactive to anti-bodies against the HCV core region

Sequence:

Gln Arg Lys Thr Lys Arg Ser Thr Asn Arg Arg

1       5       10

Sequence number: 5

Length of sequence: 19

Type of sequence: Amino acid

Topology: Normal chain

Kind of sequence: Peptide

Other information: A peptide which is reactive to anti-bodies against the HCV core region

Sequence:

Thr Gln Gln Arg Lys Thr Lys Arg Ser Thr Asn Arg

1       5       10

Arg Arg Ser Lys Asn Glu Lys

15

Sequence number: 6

Length of sequence: 17

Type of sequence: Amino acid

Topology: Normal chain

Kind of sequence: Peptide

Other information: A peptide which is reactive to anti-

bodies   against the HCV core region

Sequence:

Thr Arg Arg Leu Gly Pro Arg Leu Gly Arg Arg Pro

  1                 5               10

Ala Leu Met Ala Val

        15

Sequence number: 7

Length of sequence: 19

Type of sequence: Amino acid

Topology: Normal chain

Kind of sequence: Peptide

Other information: A peptide which is reactive to anti-

bodies against the HCV core region

Sequence:

Val Val Pro Arg Lys Gly Pro Arg Leu Gly Arg Arg

  1                 5               10

Pro Ala Leu Met Ala Val Lys

        15

Sequence number: 8

Length of sequence: 19

Type of sequence: Amino acid

Topology: Normal chain

Kind of sequence: Peptide

Other information: A peptide which is reactive to anti-
bodies against the HCV core region

Sequence:

Pro Thr Arg Arg Arg Gly Pro Arg Leu Gly Arg Arg
1 5 10

Pro Ala Leu Met Ala Val Lys
15

SEQUENCE LISTING

(1) GENERAL INFORMATION:

    (i) APPLICANT:

        (A) NAME: Eiken Kagaku Kabushiki Kaisha

        (B) STREET: 33-8, Hongo 1-chome

        (C) CITY: Bunkyo-ku

        (D) STATE: Tokyo

        (E) COUNTRY: Japan

        (F) POSTAL CODE (ZIP): Tokyo 113


        (A) NAME: Res. Foundation for Microbial Diseases of
                Osaka University

        (B) STREET: 3-1, Yamadaoka

        (C) CITY: Suita-shi

        (D) STATE: Osaka

        (E) COUNTRY: Japan

        (F) POSTAL CODE (ZIP): Osaka 565


        (A) NAME: Tanabe Seiyaku Co., Ltd.

        (B) STREET: 2-10, Dosho-machi 3-chome

        (C) CITY: Chuo-ku

        (D) STATE: Osaka-shi

        (E) COUNTRY: Japan

        (F) POSTAL CODE (ZIP): Osaka 541


    (ii) TITLE OF INVENTION: Reagents for testing for Hepatitis C and
        method of testing for the same


    (iii) NUMBER OF SEQUENCES: 9


    (iv) COMPUTER READABLE FORM:

        (A) MEDIUM TYPE: Floppy disk

        (B) COMPUTER: IBM PC compatible

        (C) OPERATING SYSTEM: PC-DOS/MS-DOS

        (D) SOFTWARE: PatentIn Release #1.0, Version #1.25 (EPO)


    (v) CURRENT APPLICATION DATA:

        APPLICATION NUMBER: EP 9290915.3

    (vi) PRIOR APPLICATION DATA:

        (A) APPLICATION NUMBER: JP 3-255524

        (B) FILING DATE: 02-OCT-1991

(vi) PRIOR APPLICATION DATA:
    (A) APPLICATION NUMBER: JP 4-068695
    (B) FILING DATE: 26-MAR-1992


(2) INFORMATION FOR SEQ ID NO: 1:


  (i) SEQUENCE CHARACTERISTICS:
    (A) LENGTH: 10 amino acids
    (B) TYPE: amino acid
    (D) TOPOLOGY: linear


(ii) MOLECULE TYPE: peptide


(ix) FEATURE:
    (D) OTHER INFORMATION: "A peptide which is reactive to antibodies against the HCV core region"


(ix) FEATURE:
    (B) LOCATION: 2
    (D) OTHER INFORMATION: /note= "Xaa is any amino acid"


(xi) SEQUENCE DESCRIPTION: SEQ ID NO: 1:


Arg Xaa Gly Pro Arg Leu Gly Arg Arg Pro
1          5          10


(2) INFORMATION FOR SEQ ID NO: 2:

  (i) SEQUENCE CHARACTERISTICS:
    (A) LENGTH: 11 amino acids
    (B) TYPE: amino acid
    (D) TOPOLOGY: linear


(ii) MOLECULE TYPE: peptide


(ix) FEATURE:
    (D) OTHER INFORMATION: "A peptide which is reactive to antibodies against the HCV core region"

(xi) SEQUENCE DESCRIPTION: SEQ ID NO: 2:

Gln Arg Lys Thr Lys Arg Ser Thr Asn Arg Arg
1                   5                   10

(2) INFORMATION FOR SEQ ID NO: 3:

    (i) SEQUENCE CHARACTERISTICS:
        (A) LENGTH: 19 amino acids
        (B) TYPE: amino acid
        (D) TOPOLOGY: linear

    (ii) MOLECULE TYPE: peptide

    (ix) FEATURE:
        (D) OTHER INFORMATION: "A peptide which is reactive to antibodies
against the HCV core region"

    (xi) SEQUENCE DESCRIPTION: SEQ ID NO: 3:

Thr Gln Gln Arg Lys Thr Lys Arg Ser Thr Asn Arg Arg Arg Ser Lys
1                   5                   10                  15

Asn Glu Lys

(2) INFORMATION FOR SEQ ID NO: 4:

    (i) SEQUENCE CHARACTERISTICS:
        (A) LENGTH: 10 amino acids
        (B) TYPE: amino acid
        (D) TOPOLOGY: linear

    (ii) MOLECULE TYPE: peptide

    (ix) FEATURE:
        (D) OTHER INFORMATION: "A peptide which is reactive to antibodies
against the HCV core region"

32

(xi) SEQUENCE DESCRIPTION: SEQ ID NO: 4:

Arg Leu Gly Pro Arg Leu Gly Arg Arg Pro
1               5                   10


(2) INFORMATION FOR SEQ ID NO: 5:

    (i) SEQUENCE CHARACTERISTICS:
        (A) LENGTH: 17 amino acids
        (B) TYPE: amino acid
        (D) TOPOLOGY: linear

    (ii) MOLECULE TYPE: peptide

    (ix) FEATURE:
        (D) OTHER INFORMATION: "A peptide which is reactive to antibodies
against the HCV core region"


    (xi) SEQUENCE DESCRIPTION: SEQ ID NO: 5:

    Thr Arg Arg Leu Gly Pro Arg Leu Gly Arg Arg Pro Ala Leu Met Ala
    1               5                   10                  15

    Val


(2) INFORMATION FOR SEQ ID NO: 6:

    (i) SEQUENCE CHARACTERISTICS:
        (A) LENGTH: 19 amino acids
        (B) TYPE: amino acid
        (D) TOPOLOGY: linear

    (ii) MOLECULE TYPE: peptide

    (ix) FEATURE:
        (D) OTHER INFORMATION: "A peptide which is reactive to antibodies
against the HCV core region"

(xi) SEQUENCE DESCRIPTION: SEQ ID NO: 6:

Val Val Pro Arg Lys Gly Pro Arg Leu Gly Arg Arg Pro Ala Leu Met
1               5                   10                  15

Ala Val Lys


(2) INFORMATION FOR SEQ ID NO: 7:

   (i) SEQUENCE CHARACTERISTICS:
      (A) LENGTH: 19 amino acids
      (B) TYPE: amino acid
      (D) TOPOLOGY: linear

   (ii) MOLECULE TYPE: peptide

   (ix) FEATURE:
      (D) OTHER INFORMATION: "A peptide which is reactive to antibodies against the HCV core region"


(xi) SEQUENCE DESCRIPTION: SEQ ID NO: 7:

Pro Thr Arg Arg Arg Gly Pro Arg Leu Gly Arg Arg Pro Ala Leu Met
1               5                   10                  15

Ala Val Lys


(2) INFORMATION FOR SEQ ID NO: 8:

   (i) SEQUENCE CHARACTERISTICS:
      (A) LENGTH: 10 amino acids
      (B) TYPE: amino acid
      (D) TOPOLOGY: linear

   (ii) MOLECULE TYPE: peptide

   (ix) FEATURE:
      (D) OTHER INFORMATION: "A peptide which is reactive to antibodies against the HCV core region"

(xi) SEQUENCE DESCRIPTION: SEQ ID NO: 8:

Arg Lys Gly Pro Arg Leu Gly Arg Arg Pro
1               5                   10


(2) INFORMATION FOR SEQ ID NO: 9:

    (i) SEQUENCE CHARACTERISTICS:
        (A) LENGTH: 10 amino acids
        (B) TYPE: amino acid
        (D) TOPOLOGY: linear

    (ii) MOLECULE TYPE: peptide

    (ix) FEATURE:
        (D) OTHER INFORMATION: "A peptide which is reactive to antibodies
against the HCV core region"


    (xi) SEQUENCE DESCRIPTION: SEQ ID NO: 9:

Arg Arg Gly Pro Arg Leu Gly Arg Arg Pro
1               5                   10


## Claims

1. Reagents for testing for hepatitis C, which comprise one or more kinds of peptides having
   Sequence 1: Arg Xaa Gly Pro Arg Leu Gly Arg Arg Pro (wherein Xaa is any given amino acid),
   or comprise multiple peptides in which peptides having the following Sequence 2 and/or Sequence 3
   are mixed with the peptide(s) of Sequence 1:
   Sequence 2: Gln Arg Lys Thr Lys Arg Ser Thr Asn Arg Arg
   Sequence 3: Thr Gln Gln Arg Lys Thr Lys Arg Ser Thr Asn Arg Arg Arg Ser Lys Asn Glu Lys.

2. Reagents for testing for hepatitis C as set forth in Claim 1 wherein Xaa in sequence 1 is an amino acid
   selected from the group consisting of Leu, Lys and Arg.

3. Reagents for testing for hepatitis C as set forth in claim 1 or 2 characterized in that the peptides are
   bound with spacers and/or vehicle proteins.

4. Reagents for testing for hepatitis C as set forth in claim 3 characterized in that the vehicle protein is
   human serum albumin.

5. A method for testing for hepatitis C by detecting antibodies which are reactive to either peptides having
   Sequence 1: Arg Xaa Gly Pro Arg Leu Gly Arg Arg Pro (wherein Xaa is any given amino acid),
   or peptides comprising multiple peptides in which peptides having the following Sequence 2 and/or
   Sequence 3 are mixed with the peptide(s) of Sequence 1:
   Sequence 2: Gln Arg Lys Thr Lys Arg Ser Thr Asn Arg Arg
   Sequence 3: Thr Gln Gln Arg Lys Thr Lys Arg Ser Thr Asn Arg Arg Arg Set Lys Asn Glu Lys.

# INTERNATIONAL SEARCH REPORT

International Application No PCT/JP92/01276

| **I. CLASSIFICATION OF SUBJECT MATTER** (if several classification symbols apply, indicate all) [6] |
|---|
| According to International Patent Classification (IPC) or to both National Classification and IPC |

Int. Cl⁵  G01N33/576

## II. FIELDS SEARCHED

| Minimum Documentation Searched [7] | |
|---|---|
| Classification System | Classification Symbols |
| IPC | G01N33/576 |

| Documentation Searched other than Minimum Documentation<br>to the Extent that such Documents are Included in the Fields Searched [8] |
|---|

Jitsuyo Shinan Koho                    1926 – 1992
Kokai Jitsuyo Shinan Koho              1971 – 1992

## III. DOCUMENTS CONSIDERED TO BE RELEVANT [9]

| Category * | Citation of Document, [11] with indication, where appropriate, of the relevant passages [12] | Relevant to Claim No. [13] |
|---|---|---|
| Y | JP, A, 3-139282 (Zaidan Hojin Handai Biseibutsubyo Kenkyukai), June 13, 1991 (13. 06. 91) | 1-5 |
| PY | JP, A, 3-284696 (Kuraray Co., Ltd.), December 16, 1991 (16. 12. 91) | 1-5 |
| PY | JP, A, 4-36185 (Kyowa Hakko Kogyo K.K.), February 6, 1992 (06. 02. 92) | 1-5 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

## IV. CERTIFICATION

| Date of the Actual Completion of the International Search | Date of Mailing of this International Search Report |
|---|---|
| October 28, 1992 (28. 10. 92) | November 17, 1992 (17. 11. 92) |
| International Searching Authority | Signature of Authorized Officer |
| Japanese Patent Office | |

Form PCT/ISA/210 (second sheet) (January 1985)